# EUROPEAN PATENT APPLICATION

(11) **EP 1 836 988 A1**
(43) Date of publication of application: **26.09.2007**
(21) Application number: 06425596.1
(22) Date of filing: 23.08.2006
(51) Int. Cl.: A61C 7/08

(54) **Orthodontic device**

(30) Priority: 24.03.2006 IT NA20060037
(71) Applicant: Aniello, Bianco, Pompei (IT)
(72) Inventor: Aniello, Bianco, Pompei (IT)

(57) **Abstract**

Instrument orthodontic able to resolve the tied up problem list to the dental overcrowdings through of the set-up dental gradual gnatologici effected according to the charts of Pound or Korkhaus.

Every set-up it corresponds to an instrument with different characteristics in how much the obtainable dental move with every single device reenters in the progressive realization of the terapy. Basides a default number of instruments doesn't exist for every single therapy, in how much the number will depend on the values emerged by the aforesaid charts.

The therapy corroborated by a medical planning is personalized according to the case. For the dental alignment the used techniques are: technical multibande, braket, expansions type Swarts

## Description

Orthodontic instrument able to resolve the tied up problem list to the dental overcrowdings through of the set-up dental gradual gnatologici effected according to the charts of Pound or Korkhaus.

Every set-up corresponds to an instrument with different characteristics in how much the obtainable dental move with every single device reenters in the progressive realization of the therapy. Besides a default number of instruments doesn't exist for every single theraphy, in how much the number will depend on the values emerged from the aforesaid charts.

The theraphy corroborated by a medical planning is personalized according to the case. For the dental alignment the used techniques are: technical multibande, braket, Swarts expansions type.

The manufactured article is worked termicamente and it is madeby various types of materials:
- Soft vinyl (you see Fig. A) of various thickness with tall absorption of energy and elevated resiliency.
- In some cases the device can be of material type: caucciù or KFO Elasto (you see Fig. A)
- Threads (you see Fig. B) modeled on the surface of the tooth vestibular and palatal way for every single tooth in arcale form; they can be type steel, nikel-titanium, memory, termomemoria of different diameters, of rectangular form (0,16mm x 0,22mm; 0,19mm x 0,25mm), square (0,16mm x 0,16mm) and round (0,155mm; 0,175mm; 0,195mm; 0,14mm; 0,16mm; 0.18mm).
- Ball hooks for the anchorage (you see Fig C) of the device inserted mesialmente, distialmente, lingually of different diameters to according to of the necessity of the case. In some cases some hooks of author Jeckson are inserted, to arrow, to butterfly.

The finalities are those of correcting the anomalies of dental overcrowdings. The dental move happens in because the instrument practices a light pressure on the dental elements. Strength is exclusively engraved on the teeth that require the correction and the alignment, in how much through this technique it is possible to exclude the pressure of the strength on some dental elements that could have a pathology paradontale avoiding the further reabsorption of the alveolar bone and resole him to her of the dental root.

These pressures are only located in some points and they are able of to practice light moves respecting the set-up performed in before according to the charts of Pound or Korkhaus. To complete the itertherapeutic they will be therefore necessary more set-up and therefore more instruments, whose number will vary according to of the gravity of the problem list.

The instrument offers the following advantages:
1. Aesthetics because few visible and removable.
2. Hygiene since it can easily be removed by the patient, being a mobile instrument, and therefore it resolves all the tied up problem list to the dental pathologies caused by the difficulties bom with the plant of a fixed instrument. Besides the instrument can be disinfected with specific products.
3. The easy management because it reduces to the least one the times of maintenance of the dentist.
4. The instrument has a small encumbrance of the oral cable and maximum O.K. from the patient.
5. Besides it has a maximum guarantee in the results.

The instrument doesn't involve any side effect.

For the dental alignment the used techniques are: technique multibande, braket, Swarts expansions type. The purpose of the instrument is that to offer an orthodontic therapy that allows an easy technical resolution of the dental overcrowding realized through the use of a series of devices, and therefore the true innovation is given by the planning and the realization of the orthodontic therapy.

The dental move, of every single tooth, it is bodily, because you have the incidence synergetic of the strength and the moment is had. Strength moves the crown movement of the tooth, while the moment is the move of the root of the tooth, that stabilizes it self in the time. The dental move is given both from the soft material with high tall absorption of energy (you see Fig. A), that from the elasticity of the thread (you see Fig. B), that can be rectangular, square or round, according to the necessity and different diameter, modeled on the vestibular and or palatal surfaces of every single tooth, and incorporated in the device.

The anchorage is realized from "ball hooks" (you see Fig C) englobed in the instrument in distal vestibular and lingual way, according to the necessity of the anchorage of the teeth, with the purpose to have a primary stability and to solicit a gradual and constant strength of every single tooth.

The therapy corroborated by a medical planning can be modified and personalized according to the case.

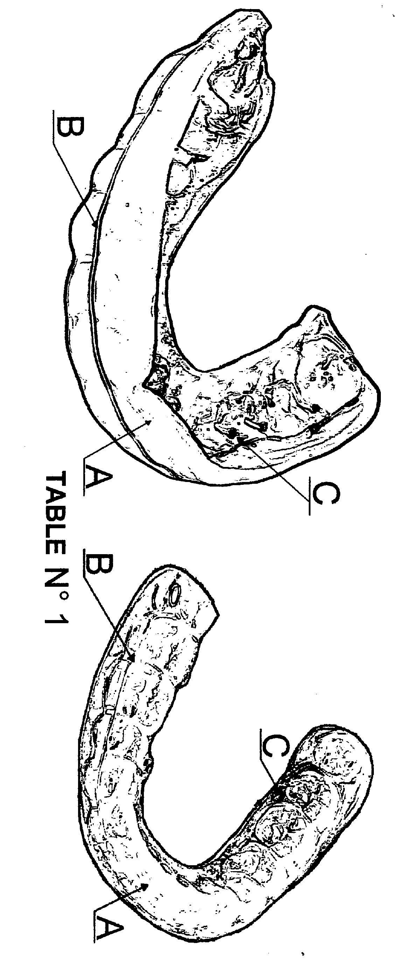

## Claims

1. You vindicates that the instrument is made of vinyl and soft material (you see Fig. A) of various thickness with high absorption of energy and elevated resiliency.

2. In some cases the device can be of material type: caoutchouc or KFO Elasto (you see Fig. To)

3. Threads (you see Fig. B) modeled on the surface of the tooth in vestibular and palatal way for every single tooth to in archal shape; they can be steel type, nikel titanium, memory, thermomemory of different diameters of rectangular (0,16mm x 0,22mm; 0,19mm x 0,25mm) square (0,16mm x 0,16mm) and round (0,155mm; 0,175mm; 0,195mm; 0,14mm; 0,16mm; 0.18mm) shape.

4. Ball hooks (you see Fig. C) for the anchorage of the device inserted in mesial and distial way, in ligual way of different diameters according to of the necessity of the case. In some cases some hooks of author Jeckson are inserted, to arrow, to butterfly.

5. It can be of all the types of color.

6. "horseshoe" type, it can be finished up from various millimeters from the collar of the tooth, it can also take the anatomical form of the same tooth.
